# EUROPEAN PATENT SPECIFICATION

(11) **EP 3 135 328 B1**
(45) Date of publication and mention of the grant of the patent: **13.06.2018**
(21) Application number: 16182607.8
(22) Date of filing: 03.08.2016
(51) Int. Cl.: A61M 5/145

(54) **SYRINGE PUMP**
SPRITZENPUMPE
POMPE DE SERINGUE

(30) Priority: 18.08.2015 JP 2015161073
(43) Date of publication of application: 01.03.2017
(73) Proprietor: Terumo Kabushiki Kaisha, Tokyo (JP)
(72) Inventor: Nakanishi, Masaru, Kanagawa, 259-0151 (JP); Maruyama, Shinji, Shizuoka, 411-0934 (JP)
(74) Representative: KIPA AB

(56) References cited:
- EP-A1- 2 301 603
- EP-A1- 2 883 560
- JP-A- 2013 135 753
- US-A1- 2003 229 311

## Description

### BACKGROUND

### Technical Field

The present invention relates to a syringe pump for pumping, to a patient, liquid medication in a syringe mounted on the syringe pump.

### Related Art

A syringe pump is used in for example an intensive-care unit (ICU) to pump, to a patient, liquid medication, such as anticancer drugs, anesthetics, chemotherapeutic agents, blood products, or nutrients, with high precision for a relatively long time. The syringe pump performs excellent and precise flow rate control of the liquid medication, compared with the other infusion pumps.

In a treatment room or operating room in which the syringe pump is used, a plurality of kinds of syringes having different capacities are prepared. A medical worker selects a syringe having a demanded capacity from the plurality of kinds of syringes, and mounts the selected syringe having a demanded capacity to the above-mentioned syringe pump.

When the syringe is mounted to a recessed mounting portion of the syringe pump, a main body flange of a syringe body is gripped, and a syringe plunger is gripped by a slider. This slider has a pair of gripping members, and an operation lever configured to fix the slider in a sliding direction or release the fixed slider.

The pair of gripping members is configured to be inclined relative to a syringe axis and separated from each other, while the medical worker presses the operation lever. The pair of gripping members are configured to grip both sides of the syringe plunger, after the medical worker releases the operation lever.

The syringe pump has a motor, and when the motor is driven, the slider gradually presses the syringe plunger toward the syringe body, the syringe plunger presses liquid medication in the syringe body and the liquid medication can be pumped to a patient through a tube (see JP 2013-135753 A).
EP2301603 discloses a syringe pump capable of holding the plungers of various types of syringes in tight contact with a slider assembly by a simple operation. The syringe pump of the invention includes grip members which are biased, on the press surface of a slider assembly, in a closing direction and towards the press surface.
US20030229311 discloses a syringe plunger driver system capable of capturing syringes of widely varying sizes and comprises a pair of asymmetric plunger retainer arms pivotally mounted to a plunger driver.
EP2883560 discloses a syringe pump in which it is possible to reduce a length of a cover member of when the cover member is contracted and to reduce a distance between a moving member of a syringe pusher driving part and a housing part of when the moving member becomes the closest to a side of the housing part and which can be downsized.

### SUMMARY

Incidentally, when the medical worker removes the syringe from the syringe pump, a plunger flange of the syringe plunger may impose an unexpected overload on the pair of gripping members. For example, when the medical worker forcibly turns the syringe while the pair of gripping members holds the plunger flange, the plunger flange imposes the overload on the pair of gripping members. Thus, the syringe forcibly turned by the medical worker upon removing the syringe from the syringe pump may break at least one of the pair of gripping members.

When the at least one of the pair of gripping members is broken, the syringe plunger cannot be pressed normally while a pair of plunger clamps of the slider grips both sides of the syringe plunger. Therefore, the slider cannot accurately press the syringe plunger, and the medication cannot be accurately pumped.

As described above, the syringe pump cannot be normally used, and thus, the syringe pump needs to be disassembled and repaired to mount a new gripping member in place of the broken gripping member.

Therefore, an object of the present invention is to provide a syringe pump configured so that when a syringe is removed from a syringe pump, a plunger flange does not impose an overload on a gripping member, and the breakage of the gripping member is prevented.

A syringe pump according to an embodiment of the present invention is a syringe pump for pumping liquid medication in a syringe mounted on the syringe pump, the syringe pump includes a mounting portion configured to mount a syringe body of the syringe thereon, and a grip mechanism unit configured to grip a syringe plunger of the syringe mounted to the mounting portion, the grip mechanism unit has at least one gripping member disposed between a plunger flange provided at the syringe plunger and the syringe body, and the gripping member has a guide portion configured to guide the plunger flange to be removed from the gripping member, upon removing the syringe body from the mounting portion.

According to the above configuration, since the at least one gripping member has the guide portion, the plunger flange is guided by the guide portion of the gripping member to be removed from the gripping member, in removal of the syringe body from the mounting portion. Therefore, when the syringe is removed from the syringe pump, the plunger flange does not impose the overload on the gripping member, and the breakage of the gripping member can be prevented. Thus, the syringe pump can be normally used, and the syringe pump does not need to be disassembled.

The guide portion is an inclined portion formed at an outer edge on a side on which the plunger flange is disposed, opposite to a side on which the syringe plunger is disposed.

According to the above configuration, the guide portion is formed at an outer edge on the side on which the plunger flange is disposed, opposite to the side on which the syringe plunger is disposed. Therefore, the guide portion being the inclined portion can guide the plunger flange to be introduced to the outer edge positioned on the side opposite to the side on which the syringe plunger is disposed, and smoothly remove the plunger flange from the gripping member. Thus, the breakage of the gripping member can be prevented.

The inclined portion has a thickness gradually reducing toward the side opposite to the side on which the syringe plunger is disposed.

According to the above configuration, the inclined portion has a thickness gradually reducing toward the side opposite to the side on which the syringe plunger is disposed. Therefore, the plunger flange can be smoothly removed from the gripping member along the gradual reduction in thickness of the inclined portion, and the breakage of the gripping member can be prevented.

Preferably, the inclined portion is provided to be at least partially positioned inside relative to an outer edge of the plunger flange, when viewed along a longitudinal axial direction of the syringe plunger being gripped by the gripping member.

Preferably, the inclined portion is provided so that an end portion of the inclined portion is positioned outside relative to the outer edge of the plunger flange, when viewed along the longitudinal axial direction of the syringe plunger gripped by the gripping member.

According to the above configuration, the end portion of the inclined portion is positioned outside relative to the outer edge of the plunger flange, when viewed along the longitudinal axial direction of the gripped syringe plunger. Therefore, while the syringe plunger is gripped by the gripping member, the end portion of the inclined portion is positioned outside relative to the outer edge of the plunger flange, so that the guide portion has an increased area for guiding the plunger flange. Thus, the plunger flange can be guided along the inclined portion and further accurately removed, without being caught by the gripping member.

The gripping member has two members of a first gripping member and a second gripping member, and the grip mechanism unit is configured to grip the syringe plunger disposed between the first gripping member and the second gripping member.

According to the above configuration, the syringe plunger is gripped from both sides by the first gripping member and the second gripping member, and thus the syringe plunger can be accurately gripped.

Preferably, an operation portion configured to turn the gripping member is provided to grip the syringe plunger.

According to the above configuration, the medical worker is only required to operate the operation portion to facilitate turning the gripping members to grip the syringe plunger or release the gripped syringe plunger.

Furthermore, a syringe pump according to an embodiment of the present invention is a syringe pump for pumping liquid medication in a syringe mounted on the syringe pump, the syringe pump includes a mounting portion configured to mount a syringe body of the syringe thereon, and a grip mechanism unit configured to grip a syringe plunger of the syringe, the grip mechanism unit has at least one gripping member disposed between a plunger flange provided at the syringe plunger and the syringe body, the gripping member has a guide portion configured to guide the plunger flange to be removed from the gripping member, upon removing the syringe body from the mounting portion, and the gripping member has a main body member having the guide portion, and a support member configured to removably mount the main body member thereto.

According to the above configuration, since the at least one gripping member has the guide portion, the plunger flange is guided by the guide portion of the gripping member to be removed from the gripping member, in removal of the syringe body from the mounting portion. Therefore, when the syringe is removed from the syringe pump, the plunger flange does not impose the overload on the gripping member, and the breakage of the gripping member can be prevented. Thus, the syringe pump can be normally used, and the syringe pump does not need to be disassembled.

In addition, the main body member of the guide portion is removably mounted to the support member. Therefore, even if the main body member having the guide portion is broken, the main body member having the guide portion can be removed from the support member and readily replaced with a new main body member, without disassembling the grip mechanism unit. Thus, the syringe pump can be normally used, and the syringe pump does not need to be disassembled.

Thus, according to an embodiment of the present invention, the syringe pump can be provided which is configured so that the plunger flange does not impose an overload on the gripping member during removal of the syringe from the syringe pump, and prevents the breakage of the gripping member.

### BRIEF DESCRIPTION OF DRAWINGS

FIG. 1 is a perspective view of a syringe pump according to a preferable first embodiment of the present invention;
FIG. 2 is a perspective view of the syringe pump illustrated in FIG. 1 viewed in a W direction;
FIGS. 3A and 3B are perspective views of examples of syringes having a plurality of kinds of sizes;
FIG. 4 is a diagram illustrating an exemplary electrical configuration in a syringe pump;
FIG. 5 is a partial perspective view of a syringe mounting portion and a syringe plunger driving unit illustrated in FIG. 2;
FIG. 6 is an enlarged partial perspective view of the syringe mounting portion and the syringe plunger driving unit illustrated in FIG. 5 which are viewed in an E direction;
FIGS. 7A and 7B are perspective views of a state in which a boot is extended maximally and a state in which a slider is ready to receive fixation of the plunger flange;
FIGS. 8A and 8B are perspective views of a state in which the boot is contracted;
FIG. 9A is a perspective view of a syringe plunger pressing member, and FIG. 9B is a perspective view of the syringe plunger pressing member which is viewed from a different angle;
FIG. 10 is a perspective view of a conventional syringe pump in which a syringe is removed by being turned in an RC direction;
FIG. 11A is a diagram illustrating an example of breakage of one gripping member in the conventional syringe pump, and FIG. 11B is a diagram illustrating an example of breakage of the other gripping member in the conventional syringe pump, for comparison;
FIG. 12 is a perspective view of a preferred embodiment of gripping members;
FIG. 13 is a perspective view of a conventional gripping member as a comparative example;
FIG. 14 is a diagram illustrating a state in which a plunger flange of a 50 mL syringe is gripped by gripping members;
FIG. 15 is a diagram illustrating a state in which a plunger flange of a 30 mL syringe is gripped by the gripping members;
FIG. 16 is a perspective view of a gripping member according to a second embodiment of the present invention; and
FIG. 17 is an exploded perspective view of the gripping member illustrated in FIG. 16.

### DETAILED DESCRIPTION

The invention is defined in claim 1. Preferred embodiments of the present invention will be described below in detail with reference to the drawings.

Note that embodiments described below are preferable specific examples of the present invention, and various technically preferable limitations are given, but the scope of the present invention is not limited to these embodiments, unless limitation of the present invention is particularly stated in the following description.

### <First embodiment>

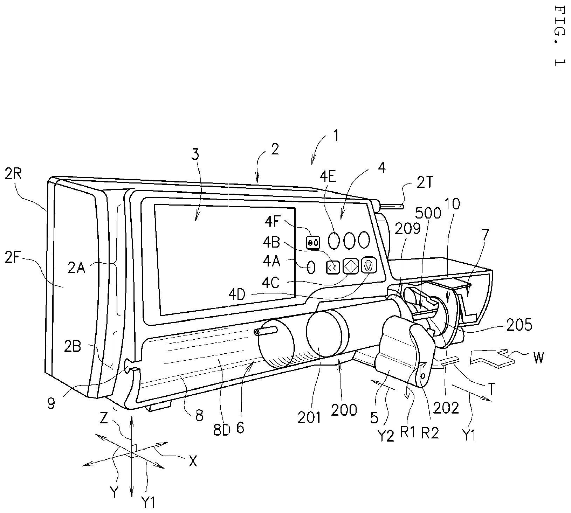

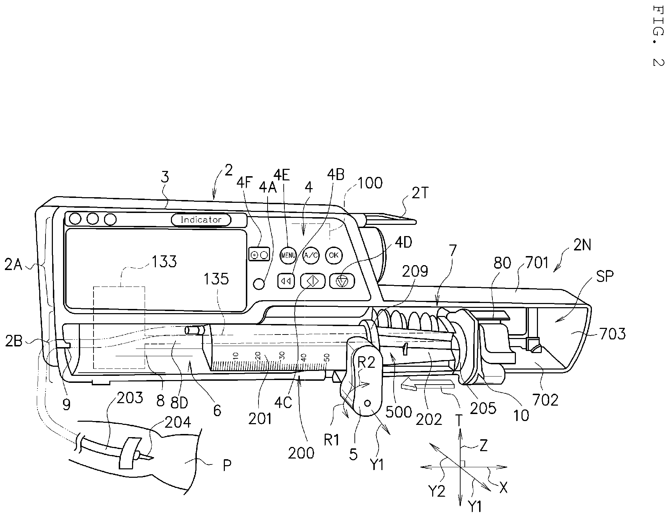

FIG. 1 is a perspective view of a syringe pump according to a first embodiment of the present invention. FIG. 2 is a perspective view of the syringe pump illustrated in FIG. 1 viewed in a W direction.

The syringe pump 1 illustrated in FIGS. 1 and 2 is a constant infusion pump which is used for example in an intensive-care unit or the like, and highly precisely performs long-time continuous infusion of liquid medication on a patient, the liquid medication including an anticancer drug, anesthetic, chemotherapeutic agent, blood product, nutrient, or the like.

As illustrated in FIGS. 1 and 2, a syringe 200 has a syringe body 201 filled with the liquid medication, for example, and the syringe pump 1 can immovably mount and fix the syringe body 201 using a clamp 5.

A syringe plunger driving unit 7 illustrated in FIG. 2 has a motor 133 and a syringe plunger pressing member 10. The motor 133 rotates a feed screw 135, and thereby the syringe plunger pressing member 10 of the syringe plunger driving unit 7 presses a syringe plunger 202 of the syringe 200 in a T direction toward the syringe body 201. Thus, the liquid medication in the syringe body 201 is accurately pumped to the patient P through a tube 203 and an indwelling needle 204, as illustrated in FIG. 2.

This syringe plunger pressing member 10 is an example of a moving member for pressing and moving the syringe plunger 202 toward the syringe body 201 while gripping the syringe plunger 202, and the syringe plunger pressing member is also referred to as slider.

As illustrated in FIG. 2, the syringe pump 1 has a housing 2, and the housing 2 is integrally molded from a molding resin material having chemical resistance. As illustrated in FIG. 1, the housing 2 is assembled by joining a front cover 2F and a rear cover 2R into a liquid-tight box body. Thus, the housing 2 has a splash-proof and drip-roof (water-proof) structure configured to prevent entrance of the liquid medication, water, or the like into the syringe pump 1, as describe later.

First, elements disposed in the housing 2 of the syringe pump 1 will be described below.

As illustrated in FIG. 2, the syringe pump 1 has the housing 2 and a handle 2T. The housing 2 has an upper portion 2A in which a display unit 3 and an operation panel unit 4 are disposed. The housing 2 has a lower portion 2B in which a syringe mounting portion 6 and a syringe plunger driving unit 7 are disposed.

Thus, a medical worker can perform pumping of the liquid medication from the syringe 200, while visually confirming the contents of information displayed in color on the display unit 3 in the upper portion 2A of the housing 2. The medical worker can operate operation buttons in the operation panel unit 4, while confirming the contents of information displayed in color on the display unit 3 in the housing 2.

The display unit 3 illustrated in FIGS. 1 and 2 is a color liquid crystal display unit (LCD) for color graphic display. The display unit 3 is disposed at an upper left position in the upper portion 2A of the housing 2, above the syringe mounting portion 6 and the syringe plunger driving unit 7. The operation panel unit 4 is disposed on the right side of the display unit 3, in the upper portion 2A of the housing 2, and in illustrated examples, the operation buttons including a pilot lamp 4A, a fast-forward switch button 4B, a start switch button 4C, a stop switch button 4D, a menu selection button 4E, a power switch 4F, and the like are disposed, in the operation panel unit 4.

The housing 2 illustrated in FIG. 2 has the upper portion 2A and the lower portion 2B. In the examples illustrated in FIGS. 1 and 2, the syringe mounting portion 6 and the syringe plunger driving unit 7 are disposed side by side along an X direction (also referred to as longitudinal axial direction). For example, the syringe 200 having a larger capacity can be selected from a plurality of kinds of syringes having different capacities, and removably fitted and mounted to the syringe mounting portion 6.

The syringe mounting portion 6 illustrated in FIGS. 1 and 2 has a storage portion 8 configured to store the syringe body 201, the clamp 5, and a main body flange gripping portion 500 configured to grip a main body flange 209 removably fitted therein. The storage portion 8 has a syringe body holding portion 8D having a recessed shape. A tube fixing portion 9 configured to removably hold the tube 203 is formed on a wall portion at a left end portion of the storage portion 8. As illustrated in FIG. 2, the tube fixing portion 9 is a groove portion configured to partially hold and fix the tube 203.

When the medical worker operates the clamp 5 to remove the syringe 200 from the syringe mounting portion 6, in FIGS. 1 and 2, for example, the clamp 5 is pulled in a Y1 direction (forward) against a force of a spring not illustrated, and further turned in an R1 direction by 90 degrees, and the clamp 5 is separated from an outer peripheral surface of the syringe body 201. Thus, the syringe body 201 can be released from the fixation by the clamp 5, and removed from the syringe body holding portion 8D of the storage portion 8, and the tube 203 can be removed from the tube fixing portion 9.

Furthermore, when the clamp 5 is operated to mount the syringe 200 to be housed in the storage portion 8 of the syringe mounting portion 6, the clamp 5 is pulled in the Y1 direction against the force of the spring not illustrated, and further turned in an R2 direction by 90 degrees, and the clamp is returned in a Y2 direction by the force of the spring. Thus, the syringe body 201 is housed in the syringe body holding portion 8D of the storage portion 8, and fixed by the clamp 5 while fitting the tube 203 in the tube fixing portion 9.

As illustrated in FIGS. 1 and 2, when the syringe body 201 is housed and mounted in the syringe body holding portion 8D of the storage portion 8, the syringe plunger 202 is disposed in the syringe plunger driving unit 7. This syringe plunger driving unit 7 has the syringe plunger pressing member 10. When a command from a control unit drives the motor 133 of FIG. 2 to rotate the feed screw 135, the syringe plunger pressing member 10 gradually presses the plunger flange 205 of the syringe plunger 202 along the T direction toward the syringe body 201. The feed screw 135 is a guide member configured to guide the syringe plunger pressing member 10 to be gradually pressed toward the storage portion 8.

Thus, the liquid medication in the syringe body 201 can be highly precisely pumped to the patient P for a relatively long time, through the tube 203 and the indwelling needle 204. The X direction, a Y direction, and a Z direction in FIGS. 1 and 2 are orthogonal to each other. The X direction represents a longitudinal axial direction of the storage portion 8 of the syringe pump 1, the Y direction represents a transverse direction of the syringe pump 1, and the Z direction represents a vertical direction of the syringe pump 1.

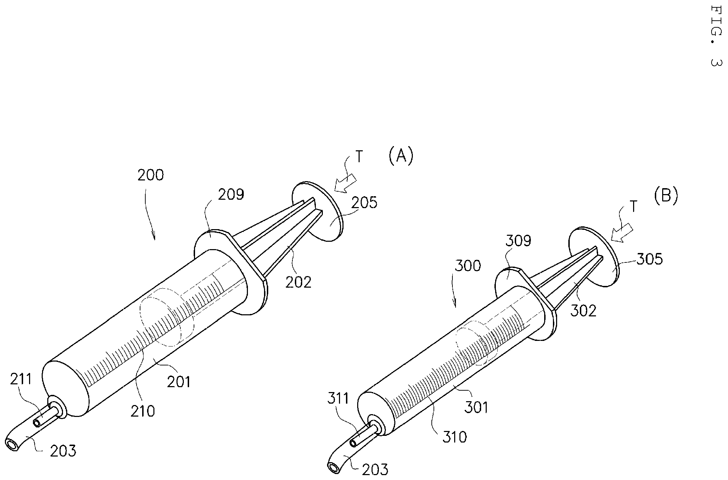

FIGS. 3A and 3B are perspective views of examples of syringes having a plurality of kinds of sizes. In FIGS. 1 and 2, as an example, the syringe 200 having the largest capacity for liquid medication is fixed.

The syringe 200 having the largest capacity for liquid medication, illustrated in FIG. 3A, has the syringe body 201 and the syringe plunger 202, the syringe body 201 has the main body flange 209, and the syringe plunger 202 has the plunger flange 205. A scale 210 for liquid medication is formed on the syringe body 201. The syringe body 201 has an outlet portion 211 to which one end portion of the tube 203 being flexible is removably mounted.

A syringe 300 having an intermediate capacity for liquid medication, illustrated in FIG. 3B, has a syringe body 301 and a syringe plunger 302, the syringe body 301 has a main body flange 309, and the syringe plunger 302 has a plunger flange 305. A scale 310 for liquid medication is formed on the syringe body 301. The syringe body 301 has an outlet portion 311 to which one end portion of the tube 203 being flexible is removably mounted.

The syringe 200 illustrated in FIG. 3A has for example a capacity for liquid medication of 50 mL, and the plunger flange 205 has a diameter of 35 mm. The syringe 300 illustrated in FIG. 3B has for example a capacity for liquid medication of 30 mL, and the plunger flange 305 has a diameter of 26 mm.

The syringe bodies 201 and 301 of the syringes 200 and 300 illustrated in FIGS. 3A and 3B have different sizes, respectively. As illustrated in FIGS. 1 and 2, the syringe bodies 201 and 301 of the syringes 200 and 300 can be fixedly housed in the syringe body holding portion 8D of the storage portion 8.

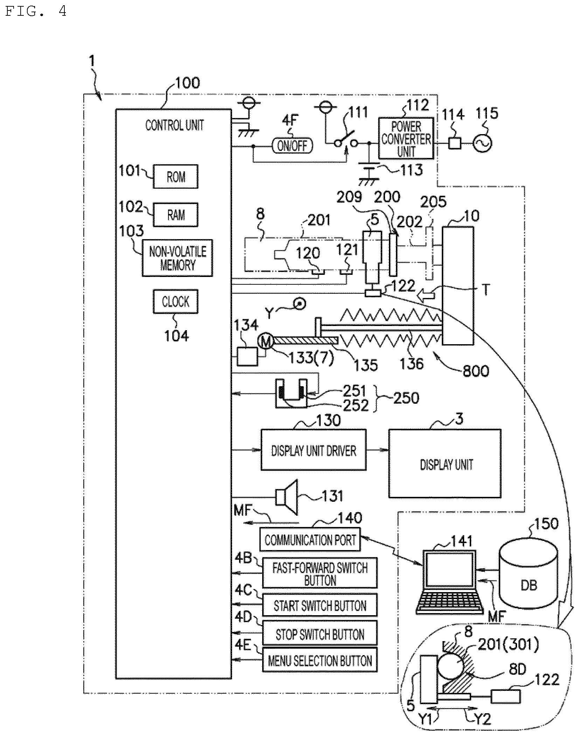

Next, an exemplary electrical configuration in the syringe pump 1 illustrated in FIGS. 1 and 2 will be described with reference to FIG. 4.

In FIG. 4, the syringe pump 1 has the control unit (computer) 100 configured to perform overall operation control. The control unit 100 is for example a one-chip microcomputer, and has a read only memory (ROM) 101, a random access memory (RAM) 102, a non-volatile memory 103, and a clock 104. The clock 104 can correct current time by predetermined operation, including obtaining current time, measurement of an elapsed time for predetermined pumping operation, measurement of a reference time for pump speed control.

A power switch 4F and a switch 111 are connected to the control unit 100 illustrated in FIG. 4. The switch 111 performs switching between a power converter unit 112 and a rechargeable battery 113 such as a lithium ion battery, and supplies power from one of the power converter unit 112 and the rechargeable battery 113, to the control unit 100. The power converter unit 112 is connected to a commercial AC power source 115 through a wall outlet 114. In FIG. 4, for example in the storage portion 8, a pair of detection switches 120 and 121 is disposed. The detection switches 120 and 121 detect whether either one of the syringe bodies of the plurality of kinds of syringes, for example, the syringe body 201 of the syringe 200 is appropriately disposed in the storage portion 8, and notify the control unit 100 of the detection result.

A potentiometer 122 as a clamp sensor, illustrated in FIG. 4, is connected to the clamp 5. The potentiometer 122 detects a movement amount of the clamp 5 moving in the Y2 direction, while clamping the syringe body 201. Then potentiometer 122 sends a detection signal to notify the control unit 100 that the syringe body 201 (301) of which capacity is clamped by the clamp 5. The control unit 100 obtains the movement amount of the clamp 5 in the Y direction based on the detection signal from the potentiometer 122, and can determine for example which syringe of the plurality of kinds of syringe bodies 201 and 301 illustrated in FIGS. 3A and 3B is mounted.

When the motor 133 of the syringe plunger driving unit 7 illustrated in FIG. 4 is driven by a motor driver 134 based on the command from the control unit 100, the motor 133 rotates the feed screw 135 to move the syringe plunger pressing member 10 in the T direction. Therefore, the syringe plunger pressing member 10 presses the syringe plunger 202 in the T direction, and accurately pumps the liquid medication in the syringe body 201 illustrated in FIG. 2 to the patient P through the tube 203 and the indwelling needle 204.

In FIG. 4, based on a command from the control unit 100, a display unit driver 130 drives the display unit 3 to display various information, notification contents, or the like thereon. A speaker 131 notifies of various notification contents based on a command from the control unit 100. The control unit 100 can perform bidirectional communication with for example an external computer 141 such as a desktop computer, through a communication port 140. The external computer 141 is connected to a liquid medication database (DB) 150, and liquid medication information MF stored in the liquid medication database 150 can be obtained by the control unit 100 through the external computer 141, and stored in the non-volatile memory 103 of the control unit 100. The control unit 100 can display the liquid medication information MF or the like on the display unit 3, based on the stored liquid medication information MF.

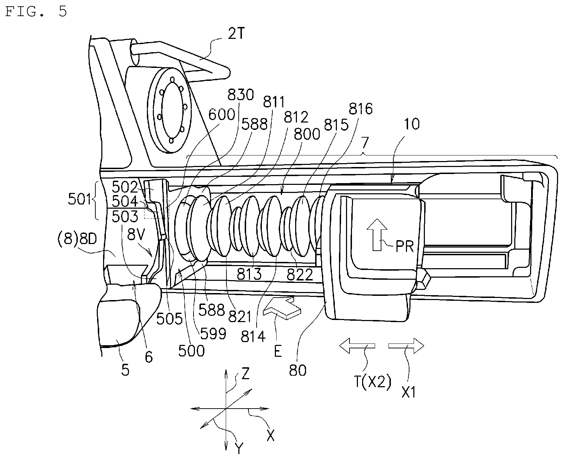

In FIG. 4, the fast-forward switch button 4B, the start switch button 4C, the stop switch button 4D, the menu selection button 4E, and the power switch 4F are electrically connected to the control unit 100. In addition, a photo-coupler sensor 250 is electrically connected to the control unit 100. The photo-coupler sensor 250 is a detector configured to detect gripping of the main body flange 209 by the main body flange gripping portion 500 (see FIG. 5). The photo-coupler sensor 250 has a light emitting element 251, and a light receiving element 252 configured to receive light from the light emitting element 251.

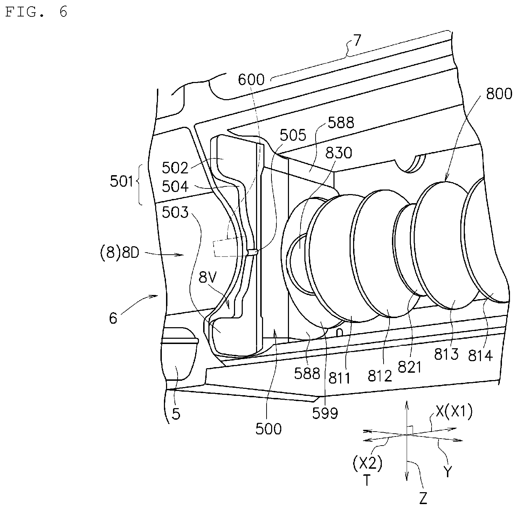

Next, a configuration of the syringe mounting portion 6 will be described in detail with reference to FIGS. 5 and 6. FIG. 5 is a partial perspective view of the syringe mounting portion 6 and the syringe plunger driving unit 7 illustrated in FIG. 2. FIG. 6 is an enlarged partial perspective view of the syringe mounting portion 6 and the syringe plunger driving unit 7 illustrated in FIG. 5 which are viewed in an E direction.

The syringe mounting portion 6 illustrated in FIG. 5 has the storage portion 8 configured to store the syringe body 201, the clamp 5, the main body flange gripping portion 500 configured to press and grip the main body flange 209 of the syringe 200 (see FIG. 3A), and a main body flange detection unit 600.

As illustrated in FIGS. 1 and 2, as an example, the syringe body 201 of the syringe 200 is set to the syringe mounting portion 6, and the syringe body 201 of the syringe 200 is fixed using the clamp 5. As illustrated in FIGS. 5 and 6, the storage portion 8 of the syringe mounting portion 6 is a recessed portion configured to house the syringe body 201, and extends along the X direction being the longitudinal axial direction of the storage portion 8. The outer peripheral surface of the syringe body 201 partially makes close contact with an inner surface of the syringe body holding portion 8D of the storage portion 8, and the rest of the outer peripheral surface of the syringe body 201 is exposed to the outside. The main body flange detection unit 600 has the photo-coupler sensor 250 illustrated in FIG. 4.

A shape of the main body flange gripping portion 500 will be described below with reference to FIGS. 5 and 6. This shape of the main body flange gripping portion 500 is exemplary only, and the main body flange gripping portion 500 is not limited to this shape. The main body flange gripping portion 500 is disposed along a plane formed by the Y direction and the Z direction.

As illustrated in FIGS. 5 and 6, the main body flange gripping portion 500 has an end portion 501 and two connection sections 588. As illustrated in FIG. 6, the end portion 501 preferably has two introduction portions 502 and 503, and a recessed portion 504 formed between the introduction portions 502 and 503. Thus, as illustrated in FIGS. 1 and 2, the medical worker can use two introduction portions 502 and 503 to readily insert the main body flange 209 of the syringe 200, between an inner surface of the main body flange gripping portion 500 and a right side surface portion 8V of the syringe body holding portion 8D, along the Y2 direction (see FIG. 1).

Consequently, the main body flange gripping portion 500 can accurately grip and fix the main body flange 209. As illustrated in FIGS. 5 and 6, a space between the connection sections 588 on both sides has a hole portion 599 having a substantially circular shape. As illustrated in FIG. 6, the hole portion 599 is formed to insert therein a boot 800 as a cover member which is described later. Therefore, when the syringe plunger 202 illustrated in FIG. 2 is pressed toward the syringe body 201 to pump the liquid medication in the syringe body 201, the boot 800 can be elastically deformed and contracted without making contact with the main body flange gripping portion 500.

Referring back to FIG. 6, while the syringe pump 1 is used, the introduction portion 502 is positioned above the introduction portion 503 in the Z direction, and the introduction portion 503 is positioned below the introduction portion 502 in the Z direction. Preferably, a small recessed portion 505 is further formed at the center position of the recessed portion 504. The small recessed portion 505 is a groove portion into which a blade portion of the syringe plunger 202 illustrated in FIG. 2 is partially inserted, while the syringe 200 is mounted. Therefore, the blade portion of the syringe plunger 202 is prevented from being on a surface of the recessed portion 504 of the main body flange gripping portion 500. Thus, the syringe 200 can be accurately fixed to a predetermined position. The syringe 300 illustrated in FIG. 3B can be also accurately fixed to the predetermined position, in addition to the syringe 200.

Next, an exemplary shape of the boot 800 as the cover member illustrated in FIGS. 5 and 6 will be simply described.

The boot 800 is a member elastically deformed and contracted upon pressing the syringe plunger 202 illustrated in FIG. 2 toward the syringe body 201 to pump the liquid medication in the syringe body 201. As illustrated in FIG. 5, the boot 800 is disposed between the right side surface portion 8V of the syringe body holding portion 8D of the storage portion 8 and the grip mechanism unit 80 of the syringe plunger pressing member 10. The boot 800 includes an elastically deformable and stretchable material, for example, rubber or plastic, and is expanded and contracted, as the syringe plunger pressing member 10 moves in an X1 direction and an X2 direction.

The boot 800 has a splash-proof structure to cover for example a machine element, such as a guide bar or a shaft 136 illustrated in FIG. 4. Thus, for example spilled liquid medication in the syringe body 201, dropped infusion arranged above the machine element, or scattered antiseptic solution, water, or the like used around the machine element can be prevented from sticking to the machine element.

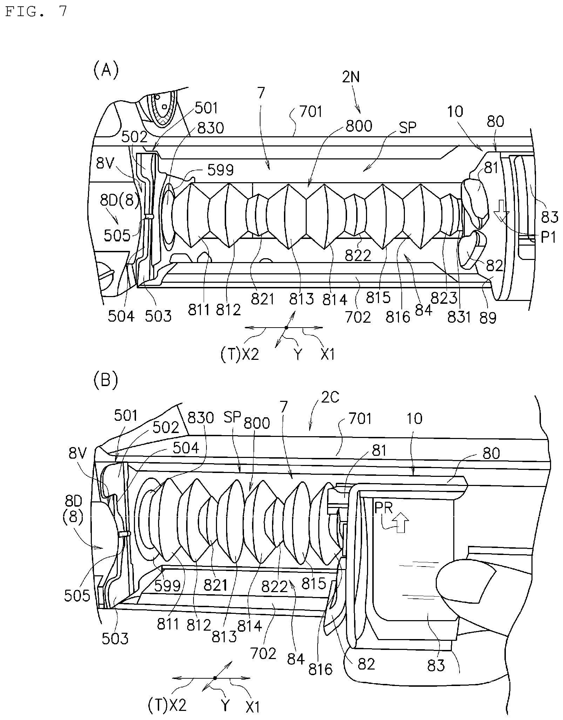

FIG. 7A illustrates a state in which the boot 800 is extended maximally, and FIG. 7B illustrates a state in which the syringe plunger pressing member 10 is ready to receive fixation of the plunger flange 205 to the syringe plunger pressing member 10.

As exemplified in FIGS. 7A and 7B, the boot 800 has for example a plurality of first projecting portions 811, 812, 813, 814, 815, and 816 of identical size, a plurality of second projecting portions 821, 822, and 823 of identical size, and right and left side connection portions 830 and 831. The second projecting portions 821, 822, and 823 have a diameter smaller than those of the first projecting portions 811, 812, 813, 814, 815, and 816.

As illustrated in FIG. 6, the left side connection portion 830 is fixed on the side of right side surface portion 8V of the syringe body holding portion 8D, through the hole portion 599 of the main body flange gripping portion 500. One second projecting portion 823 is connected to an inside surface 89 of the grip mechanism unit 80, through the right side connection portion 831.

When the syringe plunger pressing member 10 thereby moves leftward to elastically deform and contract the boot 800, the second projecting portions 821, 822, and 823 are put into the first projecting portions having a larger diameter.

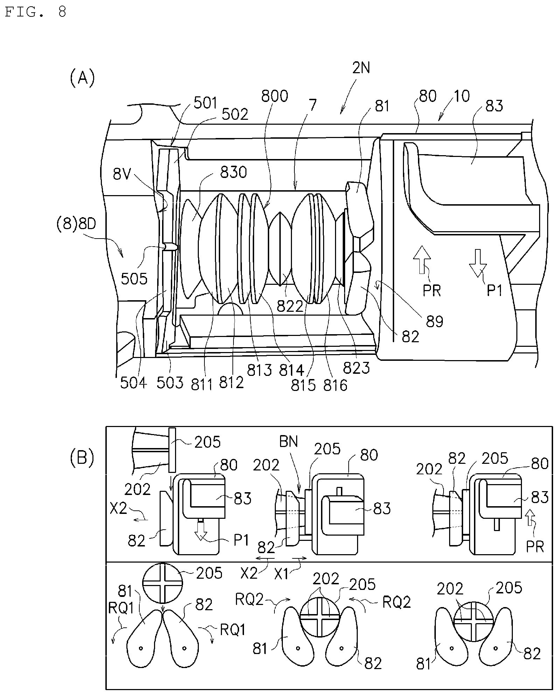

Next, an exemplary configuration of the syringe plunger driving unit 7 illustrated in FIG. 2 will be described with reference to FIGS. 7A and 7B and FIGS. 8A and 8B. FIG. 8A is a perspective view of a state in which the boot 800 is contracted.

As illustrated in FIG. 2 and FIGS. 7A and 7B, the syringe plunger driving unit 7 is housed and held in an extending portion 2N of the housing 2. The extending portion 2N is formed to extend from a lower portion of the housing 2 in the X1 direction. As illustrated in FIG. 2, the extending portion 2N has an upper side surface 701, a lower side surface 702, and a right side surface 703. The extending portion 2N has a space SP surrounded by the upper side surface 701, the lower side surface 702, the right side surface 703, and the right side surface portion 8V of the syringe body holding portion 8D of the storage portion 8 illustrated in FIGS. 7A and 7B and having been described, and the syringe plunger driving unit 7 is housed in the space SP.

When the motor 133 of the syringe plunger driving unit 7 illustrated in FIG. 4 is driven based on the command from the control unit 100, the motor rotates the feed screw 135 and moves the syringe plunger pressing member 10 in the T direction. Thus, the syringe plunger pressing member 10 presses the syringe plunger 202 in the T direction, and accurately pumps the medication in the syringe body 201 illustrated in FIG. 2 to the patient P through the tube 203 and the indwelling needle 204.

As illustrated in FIGS. 7A and 7B, the syringe plunger pressing member 10 has the grip mechanism unit 80, two gripping members 81 and 82, and an operation lever 83 as an operation portion for manually inputting an operation force for opening and closing the gripping members 81 and 82.

The gripping member 81 is an upper first gripping member, and the gripping member 82 is a lower second gripping member. The gripping members 81 and 82 can be referred to as hook. Note that, in the description of the present specification, terms "upper" and "lower" are only used for positional expressions in the present embodiment. The terms "upper" and "lower" refers to "one" and "the other" of the pair of gripping members of the grip mechanism unit 80, and there is no technical significance in direction itself represented by the terms "upper" and "lower".

The grip mechanism unit 80 is movable along a guide rail 84 of the extending portion 2N, in the X1 direction and the X2 direction (T direction). The medical worker can press the operation lever 83 with his/her finger against an urging force of the spring, in a P1 direction from an initial position illustrated in FIG. 7A, or can return the operation lever 83 lifted by the urging force of the spring, in a PR direction to the initial position, as illustrated in FIG. 7B.

As illustrated in FIG. 8B, when the medical worker presses the operation lever 83 in the P1 direction from the initial position, the gripping members 81 and 82 are moved in the X2 direction away from the grip mechanism unit 80 to have a clearance BN, and when the medical worker further presses the operation lever 83, the gripping members 81 and 82 are opened to be separated from each other in an RQ1 direction.

When the medical worker releases the operation lever 83 after fitting the plunger flange 205 of the syringe plunger 202 into the clearance BN, the operation lever 83 is returned in an RQ2 direction by spring force. Therefore, the gripping members 81 and 82 are moved in the X1 direction by the spring force not illustrated, and holds the plunger flange 205 between the gripping members 81 and 82, and the grip mechanism unit 80. The gripping members 81 and 82 are closed in the RQ2 direction, and thus the gripping members 81 and 82 can hold the syringe plunger 202 from both sides.

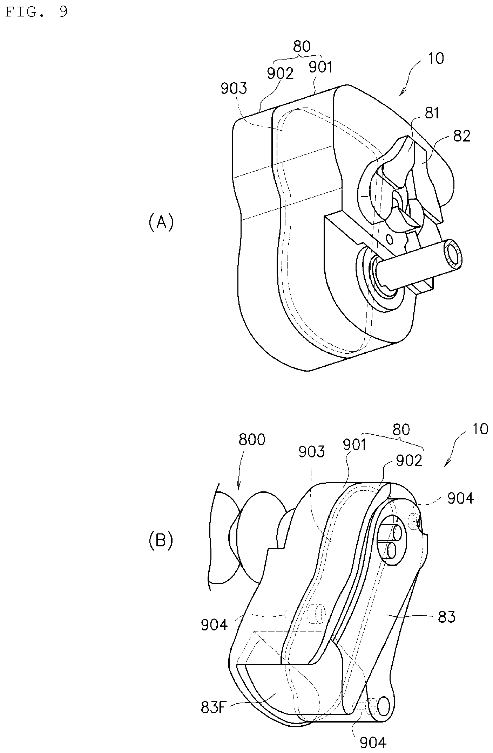

Next, an exemplary configuration of the syringe plunger pressing member 10 will be simply described with reference to FIGS. 9A and 9B.

FIG. 9A is a perspective view of the syringe plunger pressing member 10, and FIG. 9B is a perspective view of the syringe plunger pressing member 10 which is viewed from a different angle.

Referring to FIGS. 9A and 9B, the grip mechanism unit 80 of the syringe plunger pressing member 10 has a first cover 901 and a second cover 902, and a first sealing material 903 and a second sealing material 914. The second cover 902 is fixed to the first cover 901 through the first sealing material 903, with three screws 904 illustrated in FIG. 9B. The first sealing material 903 air-tightly connects a joining portion of the first cover 901 and a joining portion of the second cover 902 to form the grip mechanism unit 80 into the splash-proof structure.

In the first cover 901 and the second cover 902, mechanism elements, such as gears, of the grip mechanism unit 80 are housed. The grip mechanism unit 80 opens and closes the gripping members 81 and 82 by operating the operation lever 83, as described above.

Here, an exemplary use of the syringe pump 1 according to an embodiment of the present invention will be simply described.

The medical worker selects for example the syringe 200 from the plurality of kinds of syringes 200 and 300 illustrated in FIGS. 3A and 3B, and mounts the syringe 200 to the syringe pump 1, as illustrated in FIGS. 1 and 2. The medical worker houses the syringe body 201 in the syringe body holding portion 8D of the storage portion 8, and fixes the tube 203 being fitted into the tube fixing portion 9, by the clamp 5.

Thus, the syringe body 201 can be fixed in the syringe body holding portion 8D of the storage portion 8. Furthermore, the main body flange 209 is partially gripped between the right side surface portion 8V and the main body flange gripping portion 500. Thus, as illustrated in FIGS. 1 and 2, the main body flange 209 of the syringe 200 is accurately gripped.

As illustrated in FIGS. 7A and 7B, the medical worker presses the syringe plunger pressing member 10 in the T (X2 direction) direction while pressing the operation lever 83 in the P1 direction with his/her finger, and brings the grip mechanism unit 80 closer to the plunger flange 205 as illustrated in FIG. 8B so that two gripping members 81 and 82 of the syringe plunger pressing member 10 grips syringe plunger 202 as illustrated in FIG. 8B.

Then, when the motor 133 of the syringe plunger driving unit 7 illustrated in FIG. 4 is driven based on the command from the control unit 100, the motor rotates the feed screw 135 and moves the syringe plunger pressing member 10 in the T direction. Thus, the syringe plunger pressing member 10 presses the syringe plunger 202 in the T direction, and accurately pumps the liquid medication in the syringe body 201 illustrated in FIG. 2 to the patient P through the tube 203 and the indwelling needle 204.

Incidentally, in a conventional syringe pump, the medical worker removes a used syringe from a mounting portion of the syringe pump, at the end of pumping or upon replacement of the syringe with a new syringe.

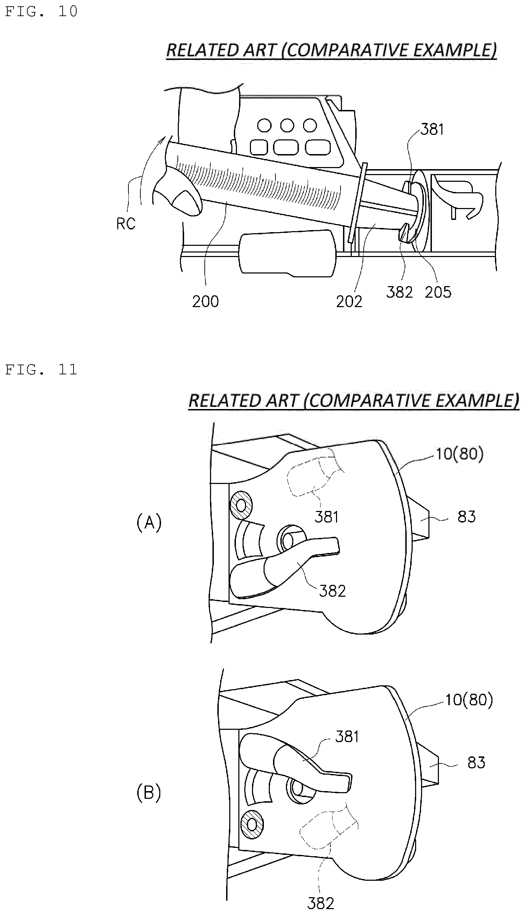

FIG. 10 is a perspective view of a conventional syringe pump in which a syringe 200 is turned in an RC direction to be removed. FIG. 11A illustrates an example of breakage of an upper gripping member 381 in the conventional syringe pump, and FIG. 11B illustrates an example of breakage of a lower gripping member 382 in the conventional syringe pump, as a comparative example with an embodiment of the present invention.

When the medical worker removes the syringe 200 from the conventional syringe pump, the plunger flange 205 may impose an unexpected overload on conventional gripping members 381 and 382.

For example, when the syringe 200, which is intended to be removed from the mounting portion of the syringe pump, is forcibly turned by the medical worker in the RC direction, while the pair of gripping members 381 and 382 holds the plunger flange, as illustrated in FIG. 10, the plunger flange 205 may impose an overload on the pair of gripping members 381 and 382.

In particular, when use of a plurality of syringe pumps are required for surgery or the like, the plurality of syringe pumps may be fixedly arranged in a vertical direction using an installation stand. In this situation, when the medical worker attempts to remove a syringe 200 from a syringe pump fixed at a higher position of the installation stand the syringe 200 may be forcibly turned downward in a direction opposite to the RC direction. At this time, as illustrated in FIG. 11A, the lower gripping member 382 remains, but the upper gripping member 381 may be broken.

In addition, when the medical worker attempts to remove the syringe 200 from the syringe pump fixed at a lower position of the installation stand, the syringe 200 may be forcibly turned in the RC direction illustrated in FIG. 10. In this situation, as illustrated in FIG. 11B, the upper gripping member 381 remains, but the lower gripping member 382 may be broken.

As described above, in the conventional syringe pump, when the medical worker forcibly turns the syringe 200, at least one of the pair of gripping members 381 and 382 may be broken.

When at least one of the pair of gripping members 381 and 382 is broken, the plunger flange 205 cannot be appropriately pressed. Therefore, the slider cannot accurately press the syringe plunger 202, and medication cannot be accurately pumped. When the conventional syringe pump cannot be used, the conventional syringe pump needs to be overhauled to mount a new gripping member.

In the syringe pump 1 according to an embodiment of the present invention, the gripping members 81 and 82 have a structure as described below to solve the problem of the conventional syringe pump.

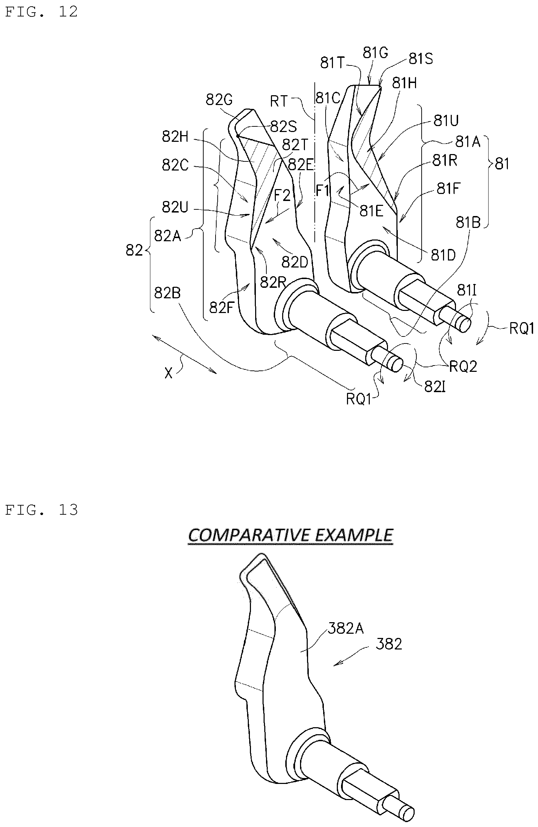

FIG. 12 is a perspective view of a preferred embodiment of the gripping members 81 and 82. FIG. 13 is a perspective view of the conventional gripping member 382 as a comparative example, for comparison with the gripping members 81 and 82 according to an embodiment of the present invention of FIG. 12.

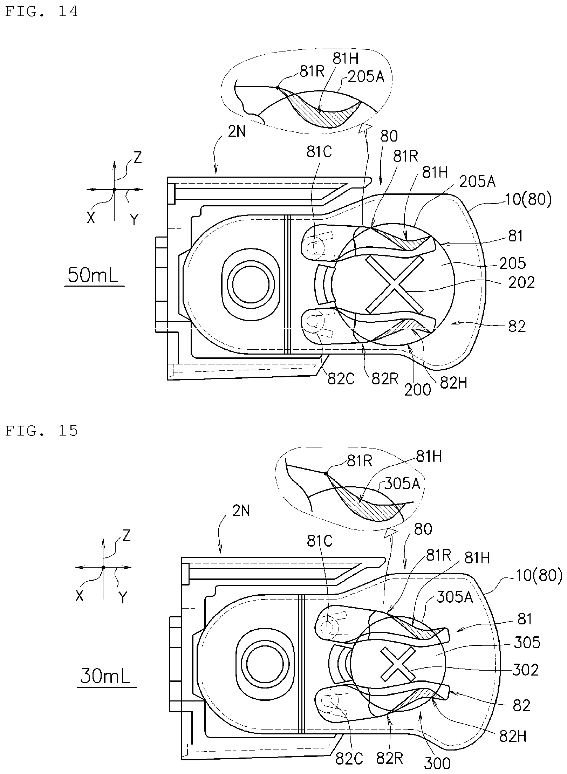

FIG. 14 is a diagram illustrating a state in which the plunger flange 205 of the syringe 200 having a capacity of 50 mL is gripped by the gripping members 81 and 82. FIG. 15 is a diagram illustrating a state in which the plunger flange 305 of the syringe 300 having a capacity of 30 mL is gripped by the gripping members 81 and 82.

FIG. 12 illustrates the upper gripping member (first gripping member) 81 and the lower gripping member (second gripping member) 82 according to an embodiment of the present invention.

The upper gripping member 81 and the lower gripping member 82 illustrated in FIG. 12 have shapes symmetrical about an axis RT. The gripping members 81 and 82 can be formed of a tough material for example polybutylene terephthalate (PBT) or polyoxymethylene (POM).

First, a configuration of the gripping member 81 is described.

As illustrated in FIG. 12, the gripping member 81 has a main body portion 81A, and a root portion 81 B integrally formed with the main body portion 81A.

First, the root portion 81B is described. The root portion 81B is formed to integrally project perpendicularly from an inner surface 81D of the main body portion 81A, and the root portion 81B is a cylindrical member. In the root portion 81B, a shaft portion 811 is fitted. The shaft portion 811 is a member made of metal such as stainless steel. The gripping member 81 is configured to turn about the shaft portion 811 to be opened in the RQ1 direction or closed in the RQ2 direction.

The main body portion 81A of the gripping member 81 has an outer surface 81C, the inner surface 81D, an inside surface 81E, and an outer edge 81F. The main body portion 81A is formed to be gradually tapered from the root portion 81B to the end portion 81G. On the inner surface 81D, an inclined portion 81H is formed as a guide portion configured to guide the plunger flange. The inner surface 81D is a surface abutting on the plunger flange.

The inclined portion 81H is formed to guide the plunger flange 205 for removal of the syringe body 201 from the mounting portion 6 illustrated in FIG. 1. In the inclined portion 81H, the plunger flange 205 slides on the gripping member 81 to be readily and smoothly removed. On the inner surface 81D, the inclined portion 81H is formed from a position apart from the root portion 81B to a position substantially in front of the end portion 81G.

However, in FIG. 12, when the inclined portion 81H is formed to the end portion 81G, the strength around the end portion 81G is undesirably reduced. Therefore, the end portion 81S on a front end side of the inclined portion 81H is formed to a position in front of the end portion 81G.

As exemplified in FIG. 14, the inclined portion 81H is formed to have a thickness gradually reducing in a F1 direction extending opposite to a side on which the syringe plunger 202 is disposed, that is, toward the outer edge 81F. Thus, the inclined portion 81H has a thickness gradually reducing toward a side opposite to the side on which the syringe plunger 202 is disposed. Therefore, since the plunger flange 205 is guided to slide along the gradual reduction in thickness of the inclined portion 81H, an abutment pressure on the plunger flange 205 is gradually reduced, and the plunger flange 205 can be readily smoothly removed from the gripping member 81. Thus, excessive force is not applied to the plunger flange removed, and breakage of the gripping member 81 can be prevented.

Furthermore, as illustrated in FIG. 14, the inclined portion 81H is provided to be at least partially positioned inside relative to an outer edge 205A of the plunger flange 205, when viewed along the longitudinal axial direction (X direction) of the syringe plunger 202 being gripped by the gripping member 81. Thus, the plunger flange 205 can be guided along the inclined portion 81H and further accurately removed, without being caught by the gripping member 81.

Furthermore, as illustrated in FIG. 14, the inclined portion 81H is provided so that an end portion 81R of the inclined portion 81H is positioned outside relative to an outer edge 205A of the plunger flange 205, when viewed along the longitudinal axial direction (X direction) of the syringe plunger 202 gripped by the gripping member 81. Thus, the plunger flange 205 can be guided along the inclined portion 81H and further accurately removed, without being caught by the gripping member 81.

While the syringe plunger 202 is gripped by the gripping members 81 and 82, the end portion 81R of the inclined portion 81H is positioned outside relative to the outer edge 205A of the plunger flange 205, so that an area for guiding the plunger flange 205 is increased in the inclined portion 81H as the guide portion. Thus, the plunger flange 205 can be guided along the inclined portion 81H and further accurately removed, without being caught by the gripping member 81.

The inclined portion 81H is represented by a hatched portion surrounded by the end portion 81R, the end portion 81S on the front end side, an inner end edge 81T, and an outer end edge 81U of the inclined portion 81H, but the hatched portion is preferably a flat surface. Therefore, the inclined portion 81H can guide the plunger flange 205 to be slid on the gripping member 81 and readily smoothly removed, in removal of the syringe body 201 from the mounting portion 6 illustrated in FIG. 1. The above also holds true for the plunger flange 305 of the syringe 300 illustrated in FIG. 15.

A shortest distance from a contact point between the syringe plunger 202 and the inside surface 81E of the gripping member 81, to the outer edge 205A of the plunger flange 205 is for example 6 mm, and a shortest distance from a contact point between the syringe plunger 302 and the inside surface 81E of the gripping member 81, to an outer edge 305A of the plunger flange 305 is for example 7 mm.

Next, a configuration of the gripping member 82 will be described.

As illustrated in FIG. 12, the gripping member 82 has a main body portion 82A, and a root portion 82B integrally formed with the main body portion 82A.

First, the root portion 82B is described. The root portion 82B is formed to integrally project perpendicularly from an inner surface 82D of the main body portion 82A, and the root portion 82B is a cylindrical member. In the root portion 82B, a shaft portion 82I is fitted. The shaft portion 82I is a member made of metal such as stainless steel. The gripping member 82 is configured to be opened in the RQ1 direction or closed in the RQ2 direction, about the shaft portion 82I.

The main body portion 82A of the gripping member 82 has an outer surface 82C, the inner surface 82D, an inside surface 82E, and an outer edge 82F. The main body portion 82A is formed to be gradually tapered from the root portion 82B to the end portion 82G. On the inner surface 82D, an inclined portion 82H is formed as a guide portion configured to guide the plunger flange.

The inclined portion 82H is formed to guide the plunger flange 205 for removal of the syringe body 201 from the mounting portion 6 illustrated in FIG. 1. In the inclined portion 82H, the plunger flange 205 slides on the gripping member 82 to be readily and smoothly removed. On the inner surface 82D, the inclined portion 82H is formed from a position apart from the root portion 82B to a position substantially in front of the end portion 82G.

However, in FIG. 12, when the inclined portion 82H is formed to the end portion 82G, the strength around the end portion 82G is undesirably reduced. Therefore, the end portion 82S on a front end side of the inclined portion 82H is formed to a position in front of the end portion 82G.

The inclined portion 82H is formed to have a thickness gradually reducing in a F1 direction extending opposite to a side on which the syringe plunger 202 is disposed, that is, toward the outer edge 82F. Thus, the inclined portion 82H has a thickness gradually reducing toward the side opposite to the side on which the syringe plunger 202 is disposed. Therefore, the plunger flange 205 can be smoothly removed from the gripping member 82 along the gradual reduction in thickness of the inclined portion 82H, and thus the breakage of the gripping member 82 can be prevented.

Furthermore, as illustrated in FIG. 14, the inclined portion 82H is provided to be at least partially positioned inside relative to an outer edge 205A of the plunger flange 205, when viewed along the longitudinal axial direction (X direction) of the syringe plunger 202 being gripped by the gripping member 82. Thus, the plunger flange 205 can be guided along the inclined portion 82H and further accurately removed, without being caught by the gripping member 82.

Furthermore, as illustrated in FIG. 14, the inclined portion 82H is provided so that an end portion 82R of the inclined portion 82H is positioned outside relative to an outer edge 205A of the plunger flange 205, when viewed along the longitudinal axial direction (X direction) of the syringe plunger 202 gripped by the gripping member 82. Thus, the plunger flange 205 can be guided along the inclined portion 82H and further accurately removed, without being caught by the gripping member 82.

While the syringe plunger 202 is gripped by the gripping members 81 and 82, an end portion 82R of the inclined portion 82H is positioned outside relative to the outer edge 205A of the plunger flange 205, so that an area for guiding the plunger flange 205 is increased in the inclined portion 82H as the guide portion. Thus, the plunger flange 205 can be guided along the inclined portion 82H and further accurately removed, without being caught by the gripping member 82.

The inclined portion 82H is represented by a hatched portion surrounded by the end portion 82R, the end portion 82S on the front end side, an inner end edge 82T, and an outer end edge 82U of the inclined portion 82H, but the hatched portion is preferably a flat surface. Therefore, the inclined portion 82H can guide the plunger flange 205 to be slid on the gripping member 82 and readily smoothly removed, in removal of the syringe body 201 from the mounting portion 6 illustrated in FIG. 1. The above also holds true for the plunger flange 305 of the syringe 300 illustrated in FIG. 15.

A shortest distance from a contact point between the syringe plunger 202 and the inside surface 82E of the gripping member 82, to the outer edge 205A of the plunger flange 205 is for example 6 mm, and a shortest distance from a contact point between the syringe plunger 302 and the inside surface 82E of the gripping member 82, to the outer edge 305A of the plunger flange 305 is for example 7 mm.

In contrast, in the gripping member 382 illustrated in FIG. 13 as the comparative example, an inner surface of the gripping member 382 only has a flat surface 382A.

As described above, in the syringe pump 1 according to the first embodiment of the present invention, when the syringe 200 (300) is removed from the syringe pump 1, the plunger flange 205 (305) does not impose the overload on the gripping members 81 and 82, and the breakage of the gripping members 81 and 82 can be prevented. Thus, the syringe pump 1 does not need to be overhauled.

### <Second embodiment>

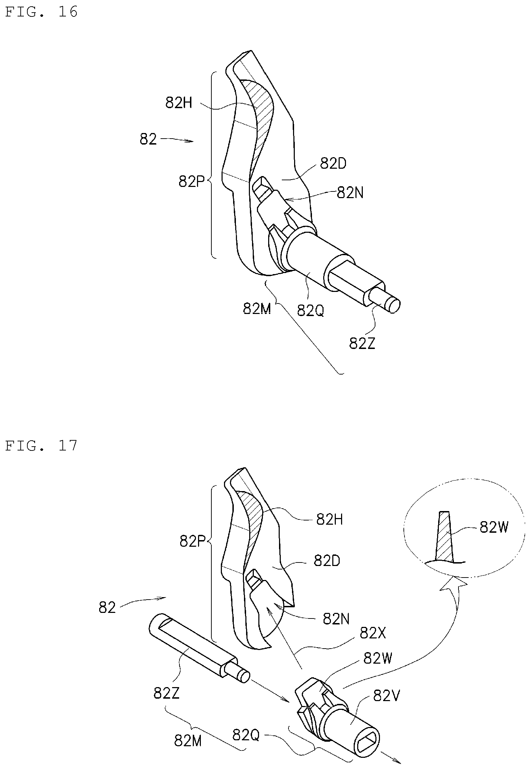

Next, a second embodiment of the present invention will be described with reference to FIGS. 16 and 17.

FIG. 16 is a perspective view of the gripping member according to a second embodiment of the present invention, and FIG. 17 is an exploded perspective view of the gripping member illustrated in FIG. 16.

FIGS. 16 and 17 representatively illustrate the lower gripping member 82, but the upper gripping member 81 is substantially the same as the lower gripping member 82 excluding its symmetrical shape with that of the lower gripping member 82, so that illustration and description of the upper gripping member 81 will be omitted.

As illustrated in FIGS. 16 and 17, the lower gripping member 82 has a main body member 82P having the inclined portion 82H, and a support member 82M. Furthermore, the support member 82M has a root portion 82Q and a shaft portion 82Z. That is, the gripping member 82 includes a total of three members of the main body member 82P, the root portion 82Q, and the shaft portion 82Z.

The main body member 82P and the root portion 82Q of the support member 82M can be formed of a tough material for example polybutylene terephthalate (PBT) or polyoxymethylene (POM). The shaft portion 82Z is made of metal such as stainless steel.

The main body member 82P has substantially the same shape as that of the main body portion 82A illustrated in FIG. 12, but the main body member 82P further has an insertion groove portion 82N. The insertion groove portion 82N is formed in the inner surface 82D along a direction toward the inclined portion 82H as the guide portion.

The root portion 82Q of the support member 82M has a cylindrical portion 82V, and an insertion piece portion 82W. The insertion piece portion 82W is removably inserted into the insertion groove portion 82N of the main body member 82P along an arrow 82X, and the main body member 82P and the support member 82M are integrated with each other. The shaft portion 82Z is integrally inserted into the cylindrical portion 82V of the root portion 82Q.

Owing to this configuration, even if the main body member 82P is broken, the main body member 82P can be removed from the root portion 82Q of the support member 82M, and only the main body member 82P can be readily replaced with a new main body member 82P. Therefore, only broken main body member 82P can be readily replaced without disassembling the grip mechanism unit 80 illustrated in FIG. 14.

Since the main body member 82P and the support member 82M are separated, the need for plastic insert molding is eliminated in manufacturing, compared with integral molding of the main body member 82P and the support member 82M. Thus, the main body member 82P and the support member 82M can be manufactured through separate continuous molding, and a risk of retention of plastic material can be reduced.

As exemplified in FIG. 17, the insertion piece portion 82W of the root portion 82Q preferably has a large thickness to be strengthened, and concentration of stress on the insertion piece portion 82W of the root portion 82Q can be avoided.

As described above, on the syringe pump 1 according to an embodiment of the present invention, the syringe 200 (300) is mounted to pump the liquid medication in the syringe. The syringe pump 1 includes the mounting portion 6 configured to mount the syringe body 201 (301) of the syringe 200 (300) thereon, and the grip mechanism unit 80 configured to grip the syringe plunger 202 (302) mounted to the mounting portion 6. The grip mechanism unit 80 has at least one of the gripping members 81 and 82 disposed between the plunger flange 205 (305) provided at the syringe plunger 202 (302) and the syringe body 201 (301). The gripping members 81 and 82 have the inclined portions 81H and 82H as the guide portion configured to guide the plunger flange 205 (305) to be removed from the gripping members 81 and 82, upon removing the syringe body 201 (301) from the mounting portion 6.

Thus, since at least one of the gripping members 81 and 82 has the inclined portion 81H or 82H as the guide portion, the plunger flange 205 (305) is guided by the inclined portion 81H or 82H as the guide portion of the gripping members 81 and 82 to be removed from the gripping members 81 and 82 in removal of the syringe body 201 (301) from the mounting portion 6. Therefore, when the syringe 200 (300) is removed from the syringe pump 1, the plunger flange 205 (305) does not impose the overload on the gripping members 81 and 82, and the breakage of the gripping members 81 and 82 can be prevented. Thus, the syringe pump 1 can be normally used, and thus the syringe pump 1 does not need to be disassembled.

The inclined portions 81H and 82H as the guide portion are the inclined portions formed at respective outer edges on the side on which the plunger flange 205 (305) is disposed, opposite to the side on which the syringe plunger 202 (302) is disposed.

Thus, the inclined portions 81H and 82H as the guide portion are formed at the outer edges 81F and 82F on the side on which the plunger flange 205 (305) is disposed, opposite to the side on which the syringe plunger 202 (302) is disposed. Thus, the inclined portions 81H and 82H as the guide portion can guide the plunger flange 205 (305) to be introduced to the outer edges 81F and 82F positioned on the side opposite to the side on which the syringe plunger 202 (302) is disposed, and smoothly remove the plunger flange from the gripping members 81 and 82. Thus, the breakage of the gripping members 81 and 82 can be prevented.

The inclined portions 81H and 82H have a thickness gradually reducing toward the side opposite to the side on which the syringe plunger 202 (302) is disposed.

Thus, the inclined portions 81H and 82H have a thickness gradually reducing toward the side on which the syringe plunger 202 (302) is disposed. That is, the inclined portions 81H and 82H have a thickness gradually reducing toward the outer edges 81F and 82F. Thus, the plunger flange 205 (305) can be smoothly removed from the gripping members 81 and 82 along the gradual reduction in thickness of the inclined portions 81H and 82H, and thus the breakage of the gripping members 81 and 82 can be prevented.

The inclined portions 81H and 82H are provided so that the end portions of the inclined portions 81H and 82H are positioned outside relative to the outer edge 205A (305A) of the plunger flange 205 (305), when viewed along the longitudinal axial direction (X direction) of the syringe plunger 202 (302) gripped by the gripping members 81 and 82.

According to the above configuration, the end portions 81R and 82R of the inclined portions 81H and 82H are positioned outside relative to the outer edge 205A (305A) of the plunger flange 205 (305), when viewed along the longitudinal axial direction (X direction) of the gripped syringe plunger 202 (302). Thus, while the syringe plunger 202 (302) is gripped by the gripping members 81 and 82, the end portions 81R and 82R of the inclined portions 81H and 82H are positioned outside relative to the outer edge 205A (305A) of the plunger flange 205 (305), so that an area for guiding the plunger flange 205 (305) is increased in the inclined portions 81H and 82H as the guide portion. Thus, the plunger flange 205 (305) can be guided along the inclined portions 81H and 82H and further accurately removed, without being caught by the gripping members 81 and 82.

The gripping member has two members of a first gripping member 81 and a second gripping member 82, and the grip mechanism unit 80 is configured to grip the syringe plunger 202 (302) disposed between the first gripping member and the second gripping member.

According to the above configuration, the syringe plunger 202 (302) is gripped from both sides by the first gripping member and the second gripping member, and thus the syringe plunger 202 (302) can be accurately gripped.

The operation lever 83 as the operation portion configured to turn the gripping members 81 and 82 is provided to grip the syringe plunger 202 (302).

According to the above configuration, the medical worker is only required to operate the operation lever 83 as the operation portion to facilitate turning the gripping members 81 and 82 to grip the syringe plunger 202 (302) or release the gripped syringe plunger 202 (302).

Further, on the syringe pump 1 according to an embodiment of the present invention, the syringe 200 (300) is mounted to pump liquid medication. The syringe pump 1 includes the mounting portion 6 configured to mount the syringe body 201 (301) of the syringe 200 (300) thereon, and the grip mechanism unit 80 configured to grip the syringe plunger 202 (302) of the syringe 200 (300). The grip mechanism unit 80 has at least one of the gripping members 81 and 82 disposed between the plunger flange 205 (305) provided at the syringe plunger 202 (302) and the syringe body 201 (301).

The gripping members 81 and 82 have the inclined portions 81H and 82H as the guide portion configured to guide the plunger flange 205 (305) to be removed from the gripping members 81 and 82, upon removing the syringe body 201 (301) from the mounting portion 6. The gripping members 81 and 82 have the main body member 82P having the inclined portions 81H and 82H as the guide portion, and the support member 82M configured to removably mount the main body member 82P thereto.

According to the above configuration, since at least one of the gripping members 81 and 82 has the inclined portion 81H or 82H as the guide portion, the plunger flange 205 (305) is guided by the inclined portion 81H or 82H as the guide portion of the gripping members 81 and 82 to be removed from the gripping members 81 and 82 in removal of the syringe body 201 (301) from the mounting portion 6.

Therefore, when the syringe 200 (300) is removed from the syringe pump 1, the plunger flange 205 (305) does not impose the overload on the gripping members 81 and 82, and the breakage of the gripping members 81 and 82 can be prevented. Thus, the syringe pump 1 can be normally used, and thus the syringe pump 1 does not need to be disassembled.

Since the main body member 82P having the guide portion is removably mounted to the support member 82M, even if for example the main body member 82P having the guide portion is broken, the main body member 82P having the guide portion is removed from the support member 82M without disassembling the grip mechanism unit 80, a new main body member 82P is mounted to the support member 82M, and the support member 82M allows easy replacement. Thus, the syringe pump 1 can be normally used, and thus the syringe pump 1 does not need to be disassembled. The gripping member 81 is also configured as described above.

The present invention is not limited to the above-mentioned embodiments, and various modifications and variations may be made within the scope of the claims. The components of the above-mentioned embodiments can be partly omitted or can be optionally combined in a manner different from the above-mentioned embodiment.

The grip mechanism unit 80 according to an embodiment of the present invention is configured to have the two gripping members 81 and 82 turning. However, the present invention is not limited to this configuration, one of the two gripping members 81 and 82 turning may be omitted, and for example a fixed gripping member, such as a projecting portion, may be formed at the syringe plunger pressing member 10, instead of the gripping member turning.

The fixed gripping member does not turn itself. In this configuration, while the plunger flange 205 (305) is fixedly pressed against the fixed gripping member, the syringe plunger 202 (302) is gripped between the fixed gripping member and one gripping member turning, by an urging force of the one gripping member turning.

As the operation portion for turning and moving the gripping members 81 and 82 in a thrust direction, the operation lever 83 is used as a manually operated power input unit. However, the operation portion is not limited to the above, and may employ an automatically operated power input unit which is operated using a motor or the like.

## Claims

1. A syringe pump (1) for pumping liquid medication in a syringe (200, 300) mounted on the syringe pump (1), comprising:
a mounting portion (6) configured to mount a syringe body (201, 301) of the syringe (200, 300) thereon; and
a grip mechanism unit (80) configured to grip a syringe plunger (202, 302) of the syringe (200, 300) mounted to the mounting portion (6),
the grip mechanism unit (80) having at least one gripping member (81, 82) configured to be disposed between a plunger flange (205, 305) provided at the syringe plunger (202, 302) and the syringe body (201, 301), the gripping member has two members, a first gripping member (81) and a second gripping member (82), and
the grip mechanism unit (80) is configured to grip the syringe plunger (202, 302) disposed between the first gripping member (81) and the second gripping member (82). the gripping member (81, 82) having a guide portion configured to guide the plunger flange (205, 305) to be removed from the gripping member (81, 82), upon removing the syringe body (201, 301) from the mounting portion (6), **characterized in that**
the guide portion is an inclined portion (81H, 82H) formed at an outer edge on a side on which the plunger flange (205, 305) is to be disposed, opposite to a side on which the syringe plunger (202, 302) is to be disposed, wherein the inclined portion (81H, 82H) has a thickness gradually reducing towards the side opposite to the side on which the syringe plunger (202, 302) is to be disposed for avoiding breaking of at least one of the first and second gripping member (81, 82) when a medical worker forcibly turns the syringe while the first and second gripping member (81, 82) hold the plunger flange such that the plunger flange imposes an overload on at least one of the first and second gripping member (81, 82).

2. The syringe pump according to claim 1, wherein
the inclined portion (81H, 82H) is configured to be at least partially positioned inside relative to an outer edge (205A, 305A) of the plunger flange (205, 305), when viewed along a longitudinal axial direction of the syringe plunger (202, 302) when being gripped by the gripping member (81, 82).

3. The syringe pump according to claim 2, wherein
the inclined portion (81H, 82H) has an end portion provided to be positioned outside relative to the outer edge (205A, 305A) of the plunger flange (205, 305), when viewed along a longitudinal axial direction of the syringe plunger (202, 302) when being gripped by the gripping member (81).

4. The syringe pump according to any of claims 1 to 3, further comprising an operation portion configured to turn the gripping member (81, 82) to grip the syringe plunger (202, 302).

5. The syringe pump according to any of claims 1 to 4, wherein
the gripping member (81, 82) has
a main body member (82P) having the guide portion, and
a support member (82M) configured to removably mount the main body member (82P) thereto.

## Patentansprüche

1. Spritzenpumpe (1) zum Pumpen von flüssigen Medikamenten in einer Spritze (200, 300), welche auf der Spritzenpumpe (1) angebracht ist, umfassend;
Montageabschnitt (6), der zum Anbringen eines Spritzenkörpers (201, 301) der Spritze (200, 300) auf demselben eingerichtet ist; und
eine Greifmechanismus-Einheit (80), die zum Greifen eines Spritzenkolbens (202, 302) der Spritze (200, 300) eingerichtet ist und die an dem Montageabschnitt (6) angebracht ist,
wobei die Greifmechanismus-Einheit (80) mindestens ein Greifbauteil (81, 82) aufweist, das zur Anordnung zwischen einer an dem Spritzenkolben (202, 302) bereitgestellten Kolbenerweiterung (205, 305) und dem Spritzenkörper (201, 301) eingerichtet ist,
das Greifbauteil zwei Bauteile, nämlich ein erstes Greifbauteil (81) und ein zweites Greifbauteil (82), aufweist, und
die Greifmechanismus-Einheit (80) zum Greifen des Spritzenkolbens (202, 302) eingerichtet ist, der zwischen dem ersten Greifbauteil (81) und dem zweiten Greifbauteil (82) angeordnet ist,
wobei das Greifbauteil (81, 82) ein Führungsbauteil aufweist, das zum Führen der Kolbenerweiterung (205, 305) eingerichtet ist, so dass sie beim Entfernen des Spritzenkörpers (201, 301) von dem Montageabschnitt (6) von dem Greifbauteil (81, 82) entfernt wird, **dadurch gekennzeichnet, dass**
es sich bei dem Führungsabschnitt um einen geneigten Abschnitt (81H, 82H) handelt, der an einer äußeren Kante auf einer Seite ausgebildet ist, auf welcher die Kolbenerweiterung (205, 305) angeordnet werden soll, und zwar gegenüber einer Seite, auf welcher der Spritzenkolben (202, 302) angeordnet werden soll, wobei der geneigte Abschnitt (81H, 82H) eine Dicke aufweist, die zu derjenigen Seite hin allmählich abnimmt, welche gegenüber der Seite liegt, auf welcher der Spritzenkolben (202, 302) angeordnet werden soll, um ein Brechen mindestens eines aus erstem und zweitem Greifbauteil (81, 82) zu verhindern, wenn ein Mitglied des medizinischen Personals die Spritze gewaltsam dreht, während das erste und zweite Greifbauteil (81, 82) die Kolbenerweiterung halten, so dass die Kolbenerweiterung eine Überlastung auf mindestens eines aus erstem und zweitem Greifbauteil (81, 82) überträgt.

2. Spritzenpumpe gemäß Anspruch 1, wobei
der geneigte Abschnitt (81H, 82H) so eingerichtet ist, dass er bei Betrachtung entlang einer längsverlaufenden axialen Richtung des Spritzenkolbens (202, 302) in Bezug auf eine äußere Kante (205A, 305A) der Kolbenerweiterung (205, 305) mindestens teilweise innen angeordnet ist, wenn er von dem Greifbauteil (81, 82) gegriffen wird.

3. Spritzenpumpe gemäß Anspruch 2, wobei
der geneigte Abschnitt (81H, 82H) einen Endabschnitt aufweist, der dazu vorgesehen ist, bei Betrachtung entlang einer längsverlaufenden axialen Richtung des Spritzenkolbens (202, 302) in Bezug auf die äußere Kante (205A, 305A) der Kolbenerweiterung (205, 305) außen angeordnet zu werden, wenn er von dem Greifbauteil (81) gegriffen wird.

4. Spritzenpumpe gemäß einem der Ansprüche 1 bis 3, ferner einen Betriebsabschnitt umfassend, der dazu eingerichtet ist, das Greifbauteil (81, 82) zu drehen, um den Spritzenkolben (202, 302) zu greifen.

5. Spritzenpumpe gemäß einem der Ansprüche 1 bis 4, wobei
das Greifbauteil (81, 82) aufweist:
ein Hauptkörperbauteil (82P), das den Führungsabschnitt aufweist, und
ein Stützbauteil (82M), das dazu eingerichtet ist, dass das Hauptkörperbauteil (82P) auf abnehmbare Weise auf demselben angebracht wird.

## Revendications

1. Pompe de seringue (1) destinée à pomper un médicament liquide dans une seringue (200, 300) montée sur la pompe de seringue (1), comprenant :
une partie de montage (6) configurée pour monter un corps de seringue (201, 301) de la seringue (200, 300) sur celle-ci ; et
une unité de mécanisme de préhension (80) configurée pour saisir un piston de seringue (202, 302) de la seringue (200, 300) montée sur la partie de montage (6),
l'unité de mécanisme de préhension (80) ayant au moins un élément de préhension (81, 82) configuré pour être disposé entre une collerette de piston (205, 305) prévue au niveau du piston de seringue (202, 302) et le corps de seringue (201, 301),
l'élément de préhension comporte deux éléments, un premier élément de préhension (81) et un deuxième élément de préhension (82), et
l'unité de mécanisme de préhension (80) est configurée pour saisir le piston de seringue (202, 302) disposé entre le premier élément de préhension (81) et le deuxième élément de préhension (82),
l'élément de préhension (81, 82) ayant une partie de guidage configurée pour guider la collerette de piston (205, 305) devant être retirée de l'élément de préhension (81, 82), lors du retrait du corps de seringue (201, 301) de la partie de montage (6), **caractérisée en ce que**
la partie de guidage est une partie inclinée (81H, 82H) formée au niveau d'un bord extérieur sur un côté sur lequel la collerette de piston (205, 305) doit être disposée, opposé à un côté sur lequel le piston de seringue (202, 302) doit être disposé, où la partie inclinée (81H, 82H) présente une épaisseur qui décroît progressivement en direction du côté opposé au côté sur lequel le piston de seringue (202, 302) doit être disposé pour éviter une rupture d'au moins l'un des premier et deuxième éléments de préhension (81, 82) lorsqu'une personne du corps médical tourne énergiquement la seringue tandis que les premier et deuxième éléments de préhension (81, 82) maintiennent la collerette de piston de telle sorte que la collerette de piston impose une surcharge sur au moins l'un des premier et deuxième éléments de préhension (81, 82).

2. Pompe de seringue selon la revendication 1, dans laquelle
la partie inclinée (81H, 82H) est configurée pour être au moins partiellement positionnée à l'intérieur par rapport à un bord extérieur (205A, 305A) de la collerette de piston (205, 305), selon une vue d'observation le long d'une direction axiale longitudinale du piston de seringue (202, 302) lorsqu'il est tenu par l'élément de préhension (81, 82).

3. Pompe de seringue selon la revendication 2, dans laquelle
la partie inclinée (81H, 82H) présente une partie d'extrémité prévue pour être positionnée à l'extérieur par rapport au bord extérieur (205A, 305A) de la collerette de piston (205, 305), selon une vue d'observation le long d'une direction axiale longitudinale du piston de seringue (202, 302) lorsqu'il est tenu par l'élément de préhension (81).

4. Pompe de seringue selon l'une quelconque des revendications 1 à 3, comprenant en outre une partie d'actionnement configurée pour pivoter l'élément de préhension (81, 82) pour saisir le piston de seringue (202, 302).

5. Pompe de seringue selon l'une quelconque des revendications 1 à 4, dans laquelle
l'élément de préhension (81, 82) comporte
un élément de corps principal (82P) intégrant la partie de guidage, et
un élément de support (82M) configuré pour monter de manière amovible l'élément de corps principal (82P) sur celui-ci.
